(19) Europäisches Patentamt
European Patent Office
Office européen des brevets

(11) EP 2 877 595 B1

(12) EUROPÄISCHE PATENTSCHRIFT

(45) Veröffentlichungstag und Bekanntmachung des Hinweises auf die Patenterteilung:
24.08.2016 Patentblatt 2016/34

(51) Int Cl.:
C12Q 1/68 (2006.01)

(21) Anmeldenummer: 13729251.2

(22) Anmeldetag: 27.05.2013

(86) Internationale Anmeldenummer:
PCT/EP2013/001564

(87) Internationale Veröffentlichungsnummer:
WO 2014/015925 (30.01.2014 Gazette 2014/05)

(54) **VERFAHREN ZUR DIFFERENZIERUNG ZWISCHEN LEBENDEN UND TOTEN ZELLEN**

METHOD FOR DIFFERENTIATING BETWEEN LIVING AND DEAD CELLS

PROCÉDÉ DE DIFFÉRENCIATION ENTRE CELLULES VIVANTES ET MORTES

(84) Benannte Vertragsstaaten:
AL AT BE BG CH CY CZ DE DK EE ES FI FR GB GR HR HU IE IS IT LI LT LU LV MC MK MT NL NO PL PT RO RS SE SI SK SM TR

(30) Priorität: 26.07.2012 DE 102012014981

(43) Veröffentlichungstag der Anmeldung:
03.06.2015 Patentblatt 2015/23

(73) Patentinhaber: Sartorius Stedim Biotech GmbH
37079 Göttingen (DE)

(72) Erfinder: SCHOLZ, Alexandra
D-37073 Göttingen (DE)

(74) Vertreter: Müller-Boré & Partner
Patentanwälte PartG mbB
Friedenheimer Brücke 21
80639 München (DE)

(56) Entgegenhaltungen:
EP-A1- 2 077 334     WO-A2-03/072805

• S. CHEN ET AL: "Rapid Detection of Viable Salmonellae in Produce by Coupling Propidium Monoazide with Loop-Mediated Isothermal Amplification", APPLIED AND ENVIRONMENTAL MICROBIOLOGY, Bd. 77, Nr. 12, 15. Juni 2011 (2011-06-15) , Seiten 4008-4016, XP055081333, ISSN: 0099-2240, DOI: 10.1128/AEM.00354-11

• YAMAZAKI WATARU ET AL: "Development of a loop-mediated Isothermal amplification assay for sensitive and rapid detection of Vibrio parahaemolyticus", BMC MICROBIOLOGY, BIOMED CENTRAL LTD, GB, Bd. 8, Nr. 1, 30. September 2008 (2008-09-30), Seite 163, XP021042376, ISSN: 1471-2180, DOI: 10.1186/1471-2180-8-163

• L. BENTSINK ET AL: "Amplification of RNA by NASBA allows direct detection of viable cells of Ralstonia solanacearum in potato", JOURNAL OF APPLIED MICROBIOLOGY, Bd. 93, Nr. 4, 1. Oktober 2002 (2002-10-01), Seiten 647-655, XP055081336, ISSN: 1364-5072, DOI: 10.1046/j.1365-2672.2002.01725.x

• J.R.C.M. VAN BECKHOVEN ET AL: "Detection of Clavibacter michiganensis subsp. sepedonicus by AmpliDet RNA, a new technology based on real time monitoring of NASBA amplicons with a molecular beacon", JOURNAL OF APPLIED MICROBIOLOGY, Bd. 93, Nr. 5, 1. November 2002 (2002-11-01), Seiten 840-849, XP055081346, ISSN: 1364-5072, DOI: 10.1046/j.1365-2672.2002.01765.x

• JUNHONG MIN ET AL: "Highly Sensitive and Specific Detection of Viable Escherichia coli in Drinking Water", ANALYTICAL BIOCHEMISTRY, Bd. 303, Nr. 2, 1. April 2002 (2002-04-01) , Seiten 186-193, XP055081380, ISSN: 0003-2697, DOI: 10.1006/abio.2002.5593

- **M. H. JOSEFSEN ET AL: "Rapid Quantification of Viable Campylobacter Bacteria on Chicken Carcasses, Using Real-Time PCR and Propidium Monoazide Treatment, as a Tool for Quantitative Risk Assessment", APPLIED AND ENVIRONMENTAL MICROBIOLOGY, Bd. 76, Nr. 15, 1. August 2010 (2010-08-01), Seiten 5097-5104, XP055081144, ISSN: 0099-2240, DOI: 10.1128/AEM.00411-10**
- **PIEPENBURG OLAF ET AL: "DNA detection using recombination proteins", PLOS BIOLOGY, PUBLIC LIBRARY OF SCIENCE, US, Bd. 4, Nr. 7, 13. Juni 2006 (2006-06-13), XP002501560, ISSN: 1544-9173, DOI: 10.1371/JOURNAL.PBIO.0040204 [gefunden am 2006-06-13]**
- **NIGEL COOK: "The use of NASBA for the detection of microbial pathogens in food and environmental samples", JOURNAL OF MICROBIOLOGICAL METHODS, Bd. 53, Nr. 2, 1. Mai 2003 (2003-05-01), Seiten 165-174, XP055081339, ISSN: 0167-7012, DOI: 10.1016/S0167-7012(03)00022-8**

SEQUENCE LISTING

<110> Sartorius Stedim Biotech GmbH

<120> Verfahren zur Differenzierung zwischen lebenden und toten Zellen

<130> S11835WO

<150> DE 10 2012 014 981.6
<151> 2012-07-26

<160> 2

<170> PatentIn version 3.5

<210> 1
<211> 35
<212> DNA
<213> Artificial sequence

<220>
<223> Primer

<400> 1

caggattaga taccctggta gtccacgccg taaac     35

<210> 2
<211> 35
<212> DNA
<213> Artificial sequence

<220>
<223> Primer

<400> 2

```
taaggggcat gatgatttga cgtcatcccc acctt
                    35
```

**Beschreibung**

Die vorliegende Erfindung betrifft ein Verfahren zur quantitativen Bestimmung von lebenden und toten Zellen in einer biologischen Probe. Das erfindungsgemäße Verfahren beruht auf der Bestimmung der DNA-Menge in der Probe mit Hilfe einer DNA-Amplifikationsreaktion, welche die Membranintegrität lebender Zellen nicht beeinträchtigt.

Im Zusammenhang mit zahlreichen Fragestellungen, beispielsweise im medizinischen, pharmazeutischen oder lebensmitteltechnologischen Bereich, ist es von Interesse, nicht nur das Vorliegen von beispielsweise kontaminierenden Bakterien in Proben nachzuweisen, sondern auch quantitative Aussagen darüber treffen zu können, ob gegebenenfalls vorliegende Bakterien lebensfähig sind. Neben traditionellen mikroskopischen und/oder mikrobiologischen Verfahren, deren Stärken nicht im Bereich hoher Sensitivität oder Schnelligkeit liegen, hat sich die molekulare Diagnostik auf Basis der Polymerasekettenreaktion (PCR) in vielfältigen Variationen zu einem Standardwerkzeug entwickelt. Mit Hilfe der "Standard-PCR" kann jedoch keine lebend/tot-Differenzierung erreicht werden, da dabei sowohl DNA lebender als auch toter Zellen amplifiziert und somit nachgewiesen wird.

Eine Gruppe PCR-gestützter Nachweisverfahren implementiert das Prinzip einer Unterscheidung zwischen genomischer DNA aus lebenden und toten Zellen unter Verwendung von Interkalationsstoffen, beispielsweise auf Basis von Ethidium- oder Propidiumaziden wie Ethidiummonoazid (EMA) oder Propidiummonoazid (PMA), welche selektiv in tote Zellen mit gestörter Membranintegrität eindringen und deren DNA durch kovalente Bindung an diese für eine PCR-Amplifikation unzugänglich machen. Nachfolgend kann selektiv nur die DNA der zuletzt lebenden Zellen vervielfältigt werden. Bekannt ist dieses Prinzip selektiver Fluoreszenzfarbstoffe, die als DNA-Interkalatoren fungieren, beispielsweise aus WO 2007/100762 A2, EP 1 992 690 A1, US 2009/0123959 A1, WO 2011/043737 A1, WO 2011/068465 A1, WO 2007/064313 A1, US 2011/0318750 A1, EP 2 077 334 A1 und CA 2768 699 A1

EMA/PMA-PCR und verwandte Verfahren finden mittlerweile Anwendung in der Lebensmitteltechnologie sowie zur Analyse von Umweltproben. Die Genauigkeit der mittels EMA/PMA-PCR erhaltenen Ergebnisse ist jedoch in vielen Fällen vermindert, beispielsweise durch die Beeinflussung von Folgeanalysen aufgrund von EMA/PMA-Rückständen, welche inhibierend auf PCR-Reaktionen wirken und im Rahmen einer DNA-Isolierung entfernt werden müssen, sowie aufgrund der unzureichenden Diskriminierung zwischen lebenden und toten Zellen. Einen weiteren Nachteil stellt die umfangreiche und zeitintensive Probenvorbereitung dar, die häufig eine Probenbehandlung und Inkubation mit dem entsprechenden DNA-Interkalator im Dunkeln, Inaktivierung des Interkalators mit Licht einer bestimmten Wellenlänge, und DNA-Isolierung zur Entfernung von Rückständen der interkalierenden Farbstoffe umfasst, bevor die eigentliche PCR, beispielsweise Realtime-PCR, da fast immer quantitative Ergebnisse von Interesse sind, durchgeführt werden kann. Darüber hinaus sind DNA-interkalierende Farbstoffe generell als potentiell mutagen anzusehen, so dass stets entsprechende Schutzmaßnahmen durch den Anwender zu treffen sind. Des Weiteren ist für eine EMA-/PMA-PCR oder ähnliche Verfahren ein kostenintensiver (Realtime)-Thermocycler notwendig, was vor allem für kleinere Labors ein Problem darstellen kann.

Aus WO 1996/014430 A1 und US 6,146,834 ist ein Verfahren zum selektiven Nachweis lebender Zellen, beispielsweise lebender Bakterien, bekannt, in dem in einem ersten Schritt die zu analysierenden lebenden Zellen durch Ausplattieren auf Agar-Medium zur Erhöhung der Zellzahl vermehrt werden. In einem zweiten Schritt werden die lebenden Zellen direkt in einer PCR-Reaktion eingesetzt, wobei kein vorgelagerter Extraktionsschritt zur Isolierung der DNA aus den Zellen durchgeführt wird. Dieses Verfahren ermöglicht einen selektiven Nachweis lebender Zellen durch den ersten Vermehrungsschritt, der ausschließlich lebende Zellen vervielfältigt und tote Zellen naturgemäß nicht vermehrt.

Nachteilig bei dem aus WO 1996/014430 A1 und US 6,146,834 bekannten Verfahren ist, dass dieses keine Quantifizierung der lebenden Zellen der Ausgangsprobe ermöglicht, da die Zellen vor der PCR durch Kultivierung vermehrt werden. Nachteilig ist weiterhin, dass eine ausreichende Vermehrung der Zellen durch Zellteilung, abhängig von den jeweiligen Zellen, mindestens mehrere Stunden bis zahlreiche Tage dauern kann, was bei zeitkritischen Analysen nicht tolerierbar ist. Außerdem werden bei WO 1996/014430 A1 und US 6,146,834 sogenannte VBNC *(viable but non-culturable bacteria)* nicht erfasst. Hierbei handelt es sich um Bakterien, die durch Umwelteinflüsse bedingt ihren Metabolismus so weit heruntergefahren haben, dass sie sich nicht mehr teilen, obwohl es sich dabei um lebensfähige Bakterien handelt. Des Weiteren ist nur ein geringer Prozentsatz aller Bakterienspezies überhaupt kultivierbar und zahlreiche Spezies sind dabei extrem anspruchsvoll und wachsen nur auf Spezialnährböden. So sind nach aktuellen Schätzungen nur 12 % aller Bakterien überhaupt kultivierbar.

WO 1999/014359 A1 offenbart ein Verfahren zur Unterscheidung zwischen lebenden und toten Mikroorganismen mittels der Insertion genetischer Marker in das Replikon der Mikroorganismen. Dieses Verfahren beruht auf der Generierung von mRNA oder cDNA aus RNA als Zielsubstrat mittels einer reversen Transkriptase oder thermostabilen Polymerase, wobei selektiv nur die genetischen Marker von lebenden Zellen detektiert werden.

Nachteilig bei dem in WO 1999/014359 A1 beschriebenen Verfahren ist, dass für die Amplifikation unter Verwendung der *"Plasmid-directed Replication"* lange Inkubationszeiten, beispielsweise über Nacht, notwendig sind. Dies bringt eine Vermehrung der Mikroorganismen mit sich, so dass anhand des Ergebnisses keine Rückschlüsse über die Anzahl der lebenden Zellen in der Ausgangsprobe gezogen werden können. Des Weiteren existieren im Rahmen einer RNA-Isolierung zahlreiche potentielle Fehlerquellen, da RNA deutlich weniger stabil ist als DNA, so dass die Effizienz im Rahmen einer RNA-Isolierung geringer ausfällt als bei einer DNA-Isolierung. Auch die Reverse-Transkriptase-PCR weist eine deutlich niedrigere Effizienz auf als Standard-PCR-Verfahren.

Aus WO 2002/052034 A1 und JP 2001/299399 A ist ein Verfahren zur Bestimmung des Verhältnisses von toten zu lebenden Zellen in einer Probe bekannt, wobei eine Reverse-Transkriptase-PCR mit einer Referenzprobe, die nur aus lebenden Zellen besteht, mit einer Referenzprobe, die nur aus toten Zellen besteht, und mit Referenzproben, die tote und lebende Zellen in bekannten, vorgegebenen Verhältnissen enthalten, durchgeführt, und eine Korrelation zwischen dem jeweiligen Mengenverhältnis zwischen toten und lebenden Zellen und dem Ergebnis der jeweiligen PCR-Reaktion erstellt wird. Dem PCR-Ergebnis einer Probe mit unbekanntem Verhältnis zwischen toten und lebenden Zellen kann dann das zugehörige Verhältnis lebender zu toten Zellen zugeordnet werden.

Nachteilig bei dem aus WO 2002/052034 A1 und JP 2001/299399 A bekannten Verfahren ist, dass die Effizienz einer RNA-Isolierung bzw. einer Reverse-Transkriptase-PCR unterhalb der Effizienz von entsprechenden DNA-basierten Verfahren liegt, so dass quantitative Ergebnisse große Fehler mit sich bringen können. Weiterhin nachteilig ist, dass die RNA-Menge in einer Zelle nicht nur davon abhängig ist, ob eine Zelle lebend oder tot ist, sondern auch von den herrschenden Umweltbedingungen. Beispielsweise wird man in lebensfähigen Endosporen kaum RNA finden, da sie ihren Metabolismus weitgehend reduziert haben. Diese Zellen würden als tot gewertet werden, da genauso wie bei toten Zellen kaum RNA nachweisbar ist.

CHEN et al. (APPLIED AND ENVIRONMENTAL MICROBIOLOGY 2011, 77(12), 4008-4016) offenbart ein quantitatives Nachweisverfahren zur Bestimmung der Zahl lebensfähiger Salmonellen in eine Probe, umfassend die Behandlung mit PMA, Inaktivierung der Zellen durch DNA-Extraktion, isothermale Amplifizierung mittels "loop-mediated isothermal amplification" (LAMP), und Nachweis durch Gelelektrophorese.

DE 10 2008 029 999 A1 offenbart ein PCR-gestütztes Verfahren unter der Bezeichnung "Splice-PCR", die sich folgendes Prinzip zunutze macht: In toten Zellen kommen sowohl die DNA-Transkription in RNA als auch die posttranskriptionelle Modifikation der RNA-Primärtranskripte zum Erliegen. Durch posttranskriptionelle Modifikation, vor allem durch das "Splicing" von Introns, entstehen Gen-spezifische Senquenzunterschiede zwischen der die Transkriptionsvorlage bildenden genomischen DNA und einer aus der modifizierten RNA durch reverse Transkription erzeugten doppelsträngigen komplementären DNA (cDNA). Da genomische DNA sowohl in lebenden als auch in toten Zellen vorliegt, gespleißte RNA jedoch nur in lebenden, kann mithilfe des beschriebenen Verfahrens aufgrund von Sequenzunterschieden das Vorliegen lebender Zellen nachgewiesen werden.

Nachteile, die sich aus DE 10 2008 029 999 A1 ergeben, sind, dass das beschriebene Verfahren keine quantitativen Ergebnisse liefert, sondern lediglich eine qualitative Aussage über das Vorhandensein lebender Zellen, da abhängig vom Lebensstadium der untersuchten Zellen große Unterschiede bezüglich der vorliegenden RNA-Menge auftreten können. Beispielsweise findet Stoffwechsel bei Sporen nur in sehr geringem Umfang statt, so dass kaum oder gar keine nachweisbare RNA vorliegt. Negativ auf die Nachweissensitivität wirkt sich auch hier das fehleranfällige Handling von RNA bei der RNA-Isolierung und der Reverse-Transkriptase-Reaktion aus. Als nachteilig ist weiterhin anzuführen, dass dieses Verfahren im Rahmen eines einzelnen Assays das Vorhandensein lebender Zellen für meist nur einen bestimmten Zielorganismus ermitteln kann, da die Intron-Exon-Struktur für ein Gen sehr stark von Organismus zu Organismus schwankt, so dass beispielsweise nicht mit universellen bakteriellen Primern gearbeitet werden kann.

Somit liegt der vorliegenden Erfindung die Aufgabe zugrunde, ein Verfahren bereitzustellen, welches eine schnelle, quantitative, möglichst sensitive und vergleichsweise preiswerte Differenzierung zwischen lebenden und toten Zellen für den Einsatz in pharmazeutischen, medizinischen, lebensmitteltechnologischen und ähnlichen Bereichen ermöglicht.

Diese Aufgabe wird durch die in den Ansprüchen gekennzeichneten Ausführungsformen gelöst.

Insbesondere betrifft ein Gegenstand der vorliegenden Erfindung ein Verfahren zur quantitativen Bestimmung von lebenden und toten Zellen in einer biologischen Probe, umfassend die Schritte:

(a) Bereitstellen eines unbehandelten Aliquots der Probe,
(b) Bereitstellen eines Aliquots der Probe, in dem alle Zellen inaktiviert wurden, wobei die Zellen durch ein Verfahren inaktiviert wurden, das einerseits zu einem vollständigen Verlust der Integrität der Zellmembranen führt und andererseits die nachfolgenden Schritte nicht behindert,
(c) Durchführen jeweils einer DNA-Amplifikationsreaktion mit den in den Schritten (a) und (b) bereitgestellten Aliquots, wobei die DNA-Amplifikationsreaktion die Membranintegrität lebender Zellen nicht beeinträchtigt und wobei die DNA-Amplifikationsreaktion eine isothermale oder nicht-isothermale Amplifikationsreaktion ist, welche bei Temperaturen unterhalb von 50 °C abläuft,
(d) Bestimmen der jeweiligen DNA-Menge in den Aliquots anhand der in Schritt (c) durchgeführten DNA-Amplifikationsreaktionen, und
(e) Bestimmen der Anzahl lebender und toter Zellen in der biologischen Probe aus den in Schritt (d) bestimmten DNA-Mengen.

Der Begriff "quantitative Bestimmung" bezieht sich dabei auf die Bestimmung der Anzahl lebender und toter Zellen in einer Probe. Die Anzahl von Zellen wird mit N bezeichnet, kann aber auch mit GE (Genomeinheiten) bezeichnet werden, da ein Genom einer Zelle entspricht. So kann beispielsweise die Anzahl lebender Zellen im Weiteren sowohl mit $N_{lebend}$ als auch mit $GE_{lebend}$ bezeichnet werden.

Die biologische Probe, in der mit Hilfe des erfindungsgemäßen Verfahrens die Anzahl lebender und toter Zellen bestimmt wird, unterliegt keinerlei besonderen Beschränkungen und umfasst jegliche Proben, in denen Zellen jeglicher Art, beispielsweise prokaryotische Zellen, vorkommen können. Biologische Proben können beispielsweise jegliche medizinische, pharmazeutische oder lebensmitteltechnologische Proben sein. Die biologische Probe und/oder die Aliquots, die daraus gebildet werden, können gegebenenfalls vor ihrer Weiterverarbeitung aufkonzentriert werden.

Das in Schritt (a) des erfindungsgemäßen Verfahrens bereitgestellte unbehandelte Aliquot der Probe kann direkt und ohne jegliche Vorbehandlung verwendet werden. Insbesondere wird dieses Aliquot keinerlei Behandlung oder Substanz ausgesetzt, welche die vorhandenen Zellen bzw. deren Membranintegrität beeinträchtigen könnte.

In dem in Schritt (b) bereitgestellten Aliquot der Probe werden alle Zellen vollständig inaktiviert. Geeignete Verfahren zur vollständigen Inaktivierung von Zellen unterliegen keinerlei besonderen Beschränkungen und sind im Stand der Technik bekannt. Sie umfassen beispielsweise jegliche Verfahren, die einerseits zu einem vollständigen Verlust der Integrität der Zellmembranen führen, und andererseits die nachfolgenden Schritte des erfindungsgemäßen Verfahrens nicht behindern. In einem konkreten Beispiel werden die Zellen durch eine Ultraschallbehandlung inaktiviert, beispielsweise für 10 Minuten. In einer speziellen Ausführungsform wird im Rahmen der Inaktivierung der Zellen die DNA der Zellen extrahiert, und in Schritt (c) des erfindungsgemäßen Verfahrens diese DNA in der entsprechenden DNA-Amplifikationsreaktion eingesetzt. Verfahren zur Extraktion von DNA aus Zellen sind im Stand der Technik bekannt.

Die in Schritt (c) des erfindungsgemäßen Verfahrens durchgeführte DNA-Amplifikationsreaktion beeinträchtigt die Membranintegrität lebender Zellen nicht. Entsprechende Verfahren unterliegen keinen besonderen Einschränkungen und sind im Stand der Technik bekannt. Bevorzugt sind dies Verfahren, die bei einer Temperatur unterhalb von 50 °C, bevorzugt zwischen 36 und 42 °C ablaufen und nicht-isothermal, bevorzugt jedoch isothermal sind, d.h. bei gleichbleibender Temperatur, durchgeführt werden. In einer bevorzugten Ausführungsform ist die DNA-Amplifikationsreaktion eine Rekombinase-Polymerase-Amplifikation (RPA). Diese ist im Stand der Technik bekannt.

Die Bestimmung der jeweiligen DNA-Menge in den Aliquots in Schritt (d) des erfindungsgemäßen Verfahrens kann mit Hilfe von Fluoreszenz-markierten Sonden, die spezifisch an die amplifizierte DNA binden, oder von DNA-bindenden Fluoreszenzfarbstoffen, wie beispielsweise "SYBR Green I", durchgeführt werden. Dabei wird die Bestimmung der jeweiligen DNA-Menge mit Hilfe eines Fluorometers, beispielsweise eines Realtime-Fluorometers, bestimmt. Entsprechende Verfahren zur Erzeugung und Bestimmung von Fluoreszenz-markierten Amplifikationsprodukten sind im Stand der Technik bekannt. In einem konkreten Beispiel kann die DNA-Amplifikationsreaktion in Anwesenheit eines DNA-bindenden Fluoreszenzfarbstoffes durchgeführt werden, wobei in regelmäßigen Abständen, beispielsweise alle 30 Sekunden, die entstehende Fluoreszenz gemessen wird. So können Fluoreszenzkurven erstellt werden, bei denen die Fluoreszenzintensität über die Zeit dargestellt wird. Die Dauer, bis die Fluoreszenzintensität einen bestimmten Schwellenwert erreicht, kann als Maß für die in der Probe bzw. in dem jeweiligen Aliquot ursprünglich vorhandene DNA-Menge verwendet werden.

Alternativ kann die Bestimmung der jeweiligen DNA-Menge in den Aliquots in Schritt (d) des erfindungsgemäßen Verfahrens mit Hilfe geeigneter Endpunktanalyseverfahren vorgenommen werden. Beispielsweise kann nach erfolgter DNA-Amplifikationsreaktion eine Gelelektrophorese durchgeführt, und ein Vergleich der Bandenstärken bzw. eine Quantifizierung derselben vorgenommen werden. Geeignete Endpunktanalyseverfahren und Verfahren zur Gelelektrophorese und Quantifizierung entsprechender Banden sind im Stand der Technik bekannt.

Bevorzugt wird die Bestimmung der Anzahl lebender und toter Zellen in der biologischen Probe in Schritt (e) des erfindungsgemäßen Verfahrens mit Hilfe einer Standardkurve durchgeführt, welche durch DNA-Amplifikationsreaktionen mit parallelen Reaktionsansätzen erhalten wird, die bekannte DNA-Konzentrationen enthalten. So können beispielsweise parallele Reaktionsansätze verwendet werden, von denen bekannt ist, wie viele Zellen der jeweils enthaltenen DNA-Menge entsprechen. So können die in den beiden im erfindungsgemäßen Verfahren verwendeten Aliquots ermittelten DNA-Mengen, bzw. die diesen entsprechenden Parameter, wie beispielsweise die Dauer, bis ein bestimmter Fluoreszenzintensitätswert während der DNA-Amplifikationsreaktion erreicht ist, mit einer entsprechenden Zellzahl korreliert werden.

Dabei erfolgt die Bestimmung der Anzahl lebender Zellen in der biologischen Probe in Schritt (e) des erfindungsgemäßen Verfahrens anhand der Formel $N_{lebend} = N_{gesamt} - N_{tot}$, bzw. anhand der äquivalenten Formel $GE_{lebend} = GE_{gesamt} - GE_{tot}$, wobei $N_{gesamt}$ bzw. $GE_{gesamt}$ anhand der in Schritt (d) bestimmten DNA-Menge in dem in Schritt (b) bereitgestellten Aliquot, und $N_{tot}$ anhand der in Schritt (d) bestimmten DNA-Menge in dem in Schritt (a) bereitgestellten Aliquot bestimmt wird. In den vorstehend genannten Formeln bezeichnet N eine Zellzahl und GE die Zahl von Genomeinheiten, wobei eine Genomeinheit einer Zelle entspricht. $N_{lebend}$ bezeichnet die Zahl der lebenden Zellen in der Probe, $N_{gesamt}$ die Gesamtzahl an Zellen, unabhängig davon, ob lebend oder tot, in der Probe, und $N_{tot}$ die Anzahl toter Zellen in der Probe.

Die der vorliegenden Erfindung zugrunde liegende Aufgabe wird dadurch gelöst, dass die quantitative Differenzierung zwischen lebenden und toten Zellen mit Hilfe einer isothermalen DNA-Amplifikationsreaktion erfolgt oder mit einer DNA-Amplifikationsreaktion, die nicht die Membranintegrität der nachzuweisenden Zellen beeinträchtigt. Es existieren verschiedene Verfahren für die isotherme DNA-Amplifikation. Ein für die hier beschriebene Anwendung geeignetes Verfahren ist die RPA, da sie bei Temperaturen unterhalb von 50 °C, bevorzugt bei einer konstanten Temperatur von 36 bis 42 °C abläuft und somit nicht zur Inaktivierung der Zellen führt. Im Vergleich inaktiviert der Denaturierungsschritt bei 95 °C in einer herkömmlichen PCR Zellen nahezu vollständig. Nichtsdestotrotz ist fast jedes DNA-Amplifikationsverfahren geeignet, das bei Temperaturen unterhalb von 50 °C, bevorzugt bei einer konstanten Temperatur von etwa 36 bis 42 °C amplifiziert und keine anderen Zell-inaktivierenden Prozesse initiiert. Dieser Temperaturbereich schafft für den Großteil aller Zellen, insbesondere aller Mikroorganismen, ideale Bedingungen, um die Lebensfähigkeit der intakten Zellen einer Probe während der Amplifikation aufrecht zu halten. So sind die Zellen auch nach der Amplifikation noch vital und es wird nur die DNA toter Zellen amplifiziert, deren Zellmembranen nicht mehr intakt sind, und somit können die Amplifikationsenzyme der RPA-Reaktion in die Zellen eindringen, um die DNA zu vervielfältigen.

Das Verfahren gemäß der vorliegenden Erfindung beinhaltet folgende Schritte: Die unbehandelte bzw. aufkonzentrierte Probe wird direkt in die isothermale Amplifikation eingesetzt, so dass nur die DNA von Zellen mit gestörter Membranintegrität für die Amplifikation zugänglich ist und somit nur die DNA toter Zellen amplifiziert wird. Zusätzlich wird parallel eine weitere isothermale Amplifikationsreaktion mit der gleichen Probe durchgeführt, wobei die Probe in diesem Fall zuvor einer DNA-Extraktion bzw. vollständigen Inaktivierung unterzogen wurde, so dass sämtliche Zellmembranen ihre Integrität verloren haben und die gesamte DNA, also sowohl die DNA der toten als auch der lebenden Zellen der Ausgangsprobe, vervielfältigt wird.

Lässt man bei der Amplifikation parallel eine Verdünnungsreihe aus quantifizierter genomischer DNA mitlaufen, so erhält man eine Standardkurve, womit sowohl die Anzahl der Genomkopien der toten Zellen als auch die Genomkopienzahl der Gesamtheit aller Zellen berechnet werden kann. Somit lässt sich die Anzahl lebender Bakterien in einer Probe wie folgt ermitteln:

$$GE_{lebend} = GE_{gesamt} - GE_{tot}$$

Gemäß einer bevorzugten Ausführungsform der Erfindung erlauben Fluoreszenz-markierte Sonden oder Fluoreszenzfarbstoffe, wie beispielsweise "SYBR Green I", die Quantifizierung und Visualisierung der Ergebnisse. Unter Verwendung der RPA werden keine Ct-Werte ("*Cycle Threshold*", Schwellenwert-Zyklus) ermittelt, wie es bei der Realtime-PCR der Fall ist, sondern die Reaktionszeiten, bis die Fluoreszenz einen bestimmten Schwellenwert überschreitet. Nichtsdestotrotz funktioniert das Prinzip, auf welchem die hier beschriebene Erfindung beruht, auch über eine Endpunktanalyse mithilfe einer Gelelektrophorese, wobei die Bandendicken des Gels ein Maß für die Menge an amplifizierter DNA bzw. an detektierten Zellen darstellen.

Weiter wird die der vorliegenden Erfindung zugrunde liegende Aufgabe dadurch gelöst, dass das Verfahren beispielsweise der isothermalen RPA innerhalb von 10 bis 30 Minuten bei konstanter Temperatur, also ohne die Notwendigkeit eines kostenintensiven Realtime-Thermocyclers, stattfindet, und eine Differenzierung zwischen lebenden und toten Zellen ermöglicht. Die Detektion der Fluoreszenzsignale kann in einem Standard-Fluorometer erfolgen, bevorzugt in einem konstant temperierbaren Realtime-Fluorometer, welches bereits für einen Bruchteil der Kosten eines Realtime-Thermocyclers erworben werden kann.

Von der Möglichkeit, dass es während der Amplifikationsreaktion zu einer Vermehrung der Zellen kommen kann, was die erhaltenen Ergebnisse verfälschen würde, kann abgesehen werden, da selbst schnell wachsende Keime eine Generationszeit von mindestens 20 Minuten in der exponentiellen Phase aufweisen, hier jedoch erst einmal eine Adaptionsphase (Lag-Phase) an die neuen "Kultivierungsbedingungen", die während der Amplifikation herrschen, durchlaufen, so dass es innerhalb dieser zeitlich sehr kurzen Amplifikationsreaktion (10 bis 30 min) zu keiner Vermehrung durch Zellteilung kommt. Bei einer weiteren bevorzugten Ausführungsform der vorliegenden Erfindung wird die Amplifikation, beispielsweise mittels RPA, bei Raumtemperatur oder noch niedrigeren Temperaturen, allerdings mit verminderter Geschwindigkeit, durchgeführt, wobei die Vermehrung der Bakterien in erwünschter Weise während der Amplifikation zusätzlich unterbunden wird.

Ein weiterer entscheidender Vorteil der Erfindung ist, dass nur eine der zwei zu amplifizierenden Proben einer DNA-Extraktion bzw. vollständigen Inaktivierung unterzogen werden muss und die zweite Probe nach der Amplifikation noch vital ist und gegebenenfalls kultiviert oder anders weiterverwendet werden kann.

Die Figuren zeigen:

Fig. 1: Zielamplikon bei 430 bp auf Agarosegel nach erfolgter RPA-Reaktion.

Fig. 2:    Standardkurve unter Verwendung quantifizierter *E. coli* DNA.

Fig. 3:    Fluoreszenzkurven der Proben 1 und 2 sowie Negativkontrollen.

Fig. 4:    Fluoreszenzkurven der Proben 3 und 4 sowie Negativkontrollen.

Fig. 5:    Standardkurve unter Verwendung quantifizierter *E. coli* DNA.

Fig. 6:    Fluoreszenzkurven vitaler und inaktivierter *E. coli* Zellen sowie Negativkontrollen.

Die vorliegende Erfindung wird anhand der folgenden, nicht-einschränkenden Beispiele näher erläutert.

Beispiel 1:

Ein Reaktionsansatz gemäß der vorliegenden Erfindung umfasst beispielsweise folgende Komponenten:

200 μl Reaktionsgefäß mit mindestens einer Rekombinase, einem SSB *(Singlestranded DNA-binding Protein),* einer DNA-Polymerase, einem *"crowding agent"* (z.B. Polyethylenglykol), einem Puffer, einem Reduktionsmittel, ATP oder einem ATP-Analogon, optional einem *"Recombinase loading Protein",* einem oder mehreren Primer(n), einer Ziel-DNA, sowie Magnesiumionen.

Es wurde mit dem RPA-Kit "TwistAmp fpg" der Firma TwistDx gearbeitet, wobei ein Teil der genannten Komponenten bereits als gefriergetrocknetes Pellet in 200-μl-Reaktionsgefäßen bereit gestellt wird. Der Anwender muss lediglich Pufferlösung, die gewünschten Primer, die Probe und Magnesiumacetat und gegebenenfalls eine Detektionssonde zufügen, um den Reaktionsansatz zu vervollständigen.

Im Rahmen von Beispiel 1 setzte sich ein 50 μl Reaktionsansatz wie folgt zusammen:

Lyophilisiertes Pellet des RPA-Kits "TwistAmp fpg"
29,5 μl Puffer des RPA-Kits "TwistAmp fpg"
420 nM Primer mit der Sequenz:
5'-CAGGATTAGATACCCTGGTAGTCCACGCCGTAAAC-3' (SEQ ID NO: 1)
420 nM Primer mit der Sequenz:
5'-TAAGGGGCATGATGATTTGACGTCATCCCCACCTT-3' (SEQ ID NO: 2)
14,2 μl Probe
12 mM Magnesiumacetat

Eine RPA-Reaktion startet erst nach Zugabe von Magnesiumacetat, da Magnesiumionen essentiell für die Aktivität der DNA-Polymerase sind.

Um eine exakt gleiche Startzeit für parallel laufende Proben zu garantieren, wird Magnesiumacetat in die Deckel der Reaktionsgefäße pipettiert, so dass mittels einer kurzen Zentrifugation das Magnesiumacetat in alle Proben gleichzeitig überführt werden kann und somit alle Reaktionen parallel starten. Ein sogenannter "Magnesium-Start" ist notwendig, da RPA-Reaktionen auch bereits bei Raumtemperatur ablaufen, wenn auch mit geringerer Effizienz.

Im Rahmen von Beispiel 1 wurde eine *Bacillus subtilis* Kultivierung (in exponentieller Phase) und eine *Escherichia coli* Kultivierung (in exponentieller Phase) 1:100 verdünnt und jeweils direkt in eine RPA-Reaktion mit der zuvor beschriebenen Reaktionsansatzzusammensetzung bei 37 °C eingesetzt. Nach einer Reaktionszeit von 30 min wurden die vollständigen Reaktionsansätze (jeweils 50 μl) auf Nähragar ausplattiert und bei 37 °C inkubiert. Innerhalb von 24 h kam es sowohl bei *E. coli* als auch bei B. *subtilis* zur Ausbildung eines Bakterienrasens.

Somit konnte anhand von Beispiel 1 gezeigt werden, dass auch nach der isothermalen Amplifikation sowohl *B. subtilis* Zellen (Gram-positiv) als auch *E. coli* Zellen (Gram-negativ) noch vital sind und keine inaktivierenden Substanzen oder Reaktionen während der Amplifikation vorhanden sind.

Beispiel 2:

Gemäß einer Ausführungsform der vorliegenden Erfindung werden die Bandendicken nach erfolgter RPA und Gelelektrophorese qualitativ beurteilt.

Als Probematerial wurde das Gram-negative Bakterium *E. coli* sowohl in der exponentiellen Phase und als auch nach vollständiger Inaktivierung mittels Ultraschall eingesetzt. Als Vertreter einer Gram-positiven Spezies kam B. *subtilis* zum Einsatz, in der stationären Phase und nach vollständiger Inaktivierung.

Die Versuchsbedingungen waren wie folgt gewählt:

Amplifikation 30 min bei 37 °C mit folgenden Komponenten pro 50 μl Reaktionsansatz:

Lyophilisiertes Pellet des RPA-Kits "TwistAmp fpg"
29,5 μl Puffer des RPA-Kits "TwistAmp fpg"
420 nM Primer mit der Sequenz:
5'-CAGGATTAGATACCCTGGTAGTCCACGCCGTAAAC-3' (SEQ ID NO: 1)
420 nM Primer mit der Sequenz:
5'-TAAGGGGCATGATGATTTGACGTCATCCCCACCTT-3' (SEQ ID NO: 2)
14,2 μl Probe
12 mM Magnesiumacetat

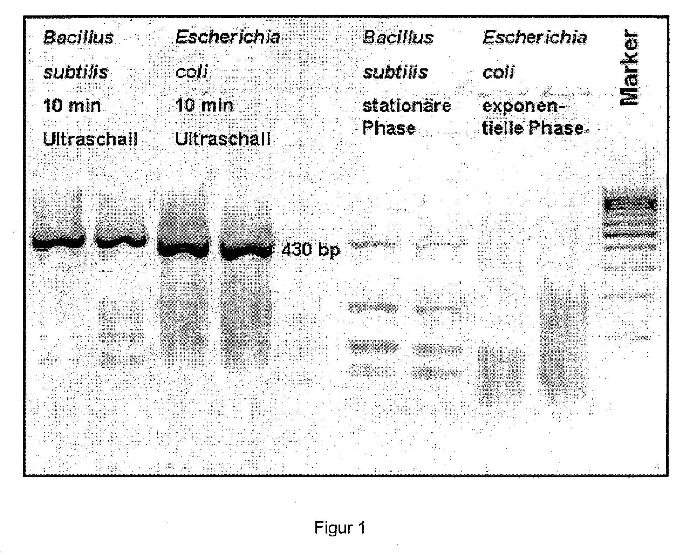

Nach erfolgter Amplifikationsreaktion wurden die vollständigen Reaktionsansätze mithilfe einer auf Siliciumdioxid basierenden Membran zur DNA-Isolierung in Anwesenheit chaotroper Verbindungen für die anschließende Agarose-Gelelektrophorese aufgereinigt (Fig. 1).

Im Rahmen von Beispiel 2 konnte gezeigt werden, dass eine *E. coli* Kultivierung in der exponentiellen Phase zu keiner DNA-Amplifikation führt (keine Banden auf dem Agarosegel), da es sich um eine im Idealfall zu 100% vitale Kultivierung handelt. Weiterhin konnte gezeigt werden, dass eine *B. subtilis* Kultivierung in der stationären Phase zu dünnen DNA-Banden (Amplikon bei 430 bp) führt, da es in der stationären Phase bereits zum Absterben von Zellen kommt. Zuvor inaktivierte B. subtilis und E. coli Proben (10 min Ultraschall) resultierten hingegen erwartungsgemäß in deutlich dickeren DNA-Banden bei 430 bp.

Beispiel 3:

Folgende Proben wurden jeweils in Dreifachbestimmung vergleichend mittels RPA analysiert, um das Quantifizierungspotential des erfindungsgemäßen Verfahrens aufzuzeigen:

1. *E. coli* (exponentielle Phase) wurde 10 min vollständig mittels Ultraschall inaktiviert und anschließend direkt in die RPA-Reaktion eingesetzt.

2. *E. coli* (exponentielle Phase) wurde 10 min vollständig mittels Ultraschall inaktiviert, die DNA mit einem Silicamembran-basierten Extraktionskit isoliert und anschließend in die RPA-Reaktion eingesetzt.

3. *E. coli* (exponentielle Phase) wurde mit einem DNA-Spike von 6,5 x $10^6$ Genomeinheiten (GE) pro RPA-Reaktion einer quantifizierten genomischen E. *coli* DNA versetzt und anschließend isothermal amplifiziert.

4. Wasser (PCR-Grade) wurde mit einem DNA-Spike von 6,5 x $10^6$ Genomeinheiten (GE) einer quantifizierten genomischen *E. coli* DNA pro RPA-Reaktion versetzt und anschließend isothermal amplifiziert.

Eine Standardkurve mit quantifizierter genomischer DNA und Negativkontrollen (Kontrolle ohne Template; Zugabe von "PCR-Grade"-Wasser anstatt der Probe) wurden parallel in der RPA mitlaufen gelassen. Die Reaktion lief bei 37 °C über einen Zeitraum von 30 min hinweg. Alle 30 Sekunden wurden Fluoreszenzdaten aufgezeichnet.

Ein 50 μl-Reaktionsansatz enthielt folgende Komponenten:

Lyophilisiertes Pellet des RPA-Kits "TwistAmp fpg"
29,5 μl Puffer des RPA-Kits "TwistAmp fpg"
420 nM Primer mit der Sequenz:
5'-CAGGATTAGATACCCTGGTAGTCCACGCCGTAAAC-3' (SEQ ID NO: 1)
420 nM Primer mit der Sequenz:
5'-TAAGGGGCATGATGATTTGACGTCATCCCCACCTT-3' (SEQ ID NO: 2)
1 μl "SYBR Green I" (1:50.000 verdünnt)
13,2 μl Probe
12 mM Magnesiumacetat

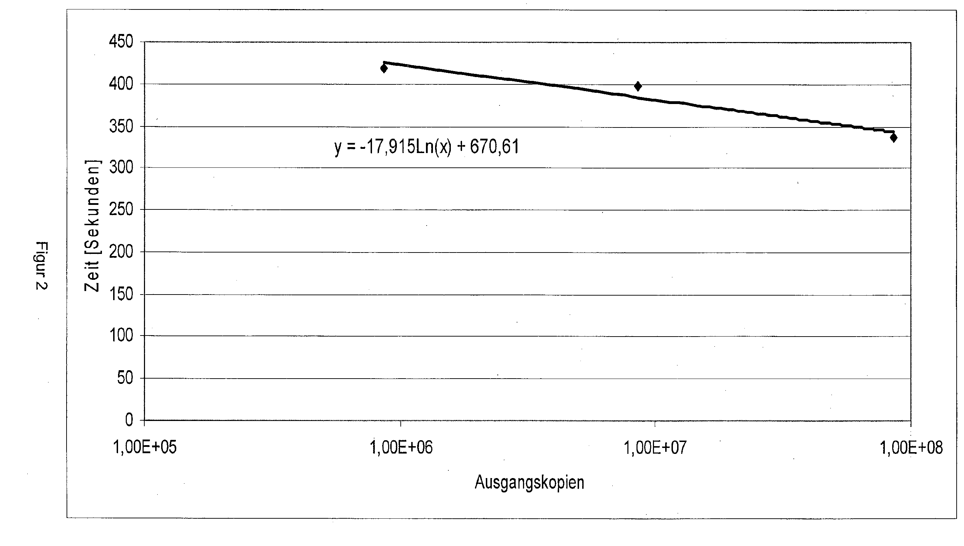

Die Standardkurve sowie die entsprechenden Werte sind in Figur 2 und Tabelle 1 gezeigt.

Tabelle 1: Werte der Standardkurve unter Verwendung quantifizierter *E. coli* DNA

| Quantifizierter DNA-Standard | Zeit bis Fluoreszenzzunahme [Sekunden] (Mittelwert, n=2) | Konzentration [Kopien/Reaktion] |
|---|---|---|
| *E.coli* Standard 1:10 verdünnt | 337 | $8,58 \times 10^7$ |
| *E.coli* Standard 1:100 verdünnt | 398 | $8,58 \times 10^6$ |
| *E.coli* Standard 1:1000 verdünnt | 419 | $8,58 \times 10^5$ |

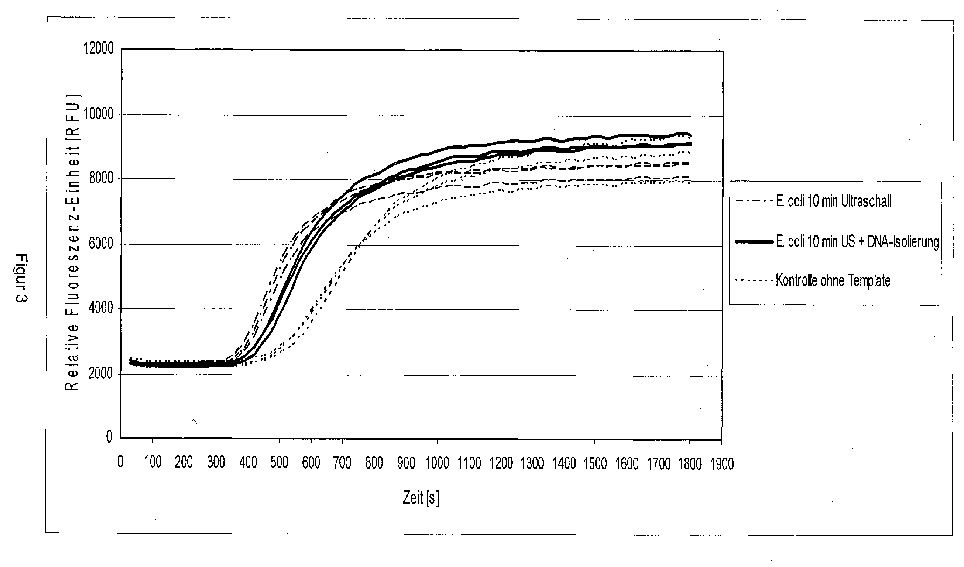

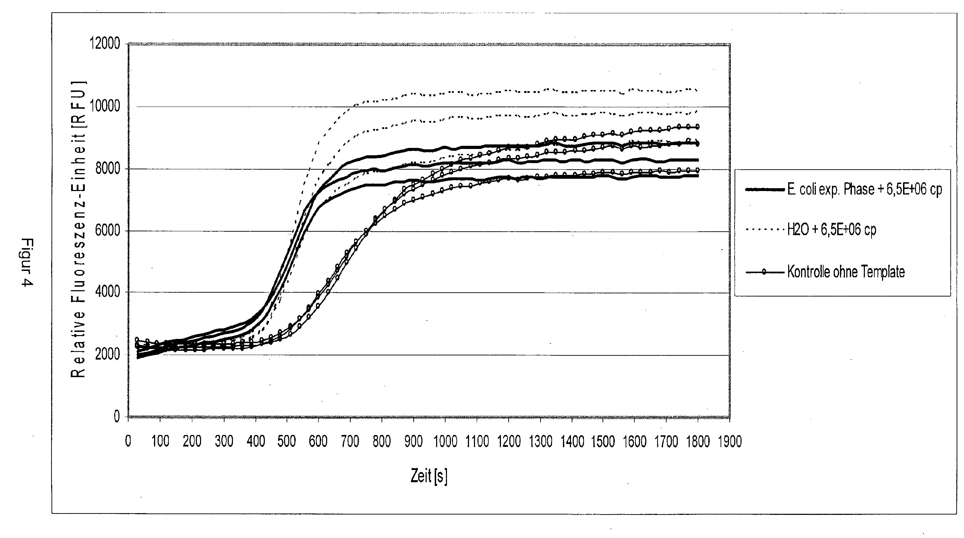

Die für die Proben 1 bis 4 erhaltenen Werte und Fluoreszenzkurven sind in den Figuren 3 und 4, sowie in Tabelle 2 gezeigt.

Tabelle 2: Ermittelte Konzentrationen der Genomeinheiten pro Reaktion

| Probe | Zeit bis Fluoreszenzzunahme [Sekunden] | Konzentration [Kopien/Reaktion] |
|---|---|---|
| *E. coli* 10 min Ultraschall (1) | 378 | $1,62 \times 10^7$ |
| | 368 | |
| | 375 | |
| *E. coli* 10 min Ultraschall + DNA-Isolierung (2) | 420 | $2,35 \times 10^6$ |
| | 403 | |
| | 404 | |
| *E. coli* exponentielle Phase + $6,5 \times 10^6$ GE (3) | 408 | $1,62 \times 10^6$ |
| | 416 | |
| | 422 | |
| $H_2O$ + $6,5 \times 10^6$ GE (4) | 398 | $2,77 \times 10^6$ |
| | 412 | |
| | 408 | |
| Kontrolle ohne Template | 509 | $2,06 \times 10^4$ |
| | 491 | |
| | 486 | |

Die Ergebnisse in Tabelle 2 sowie in den Figuren 3 und 4 zeigen, dass die vorliegende Erfindung es ermöglicht, quantitativ zwischen lebenden und toten Mikroorganismen zu unterscheiden.

Zwischen den Proben 1 und 2 ist hinsichtlich Kurvenverlauf und ermittelter Konzentration an Genomeinheiten nur ein geringer Unterschied ersichtlich, der sich durch die DNA-Verluste im Rahmen der bei Probe 2 durchgeführten Silicamembran-basierten DNA-Isolierung erklären lässt. Dieser Ansatz hat jedoch gezeigt, dass sich die DNA von Bakterien mit gestörter Mebranintegrität vollständig mittels RPA vervielfältigen lässt.

Die Kurvenverläufe von Probe 3 und 4 (Fig. 4), sowie die ermittelten Genomeinheiten-Konzentrationen (Kopien/Reaktion; Tabelle 2) sind nahezu identisch, was verdeutlicht, dass vitale Zellen während einer RPA-Reaktion intakt bleiben und deren DNA nicht amplifiziert wird.

Die Negativkontrollen ("Kontrolle ohne Template" in den Fig. 3 und 4, sowie in Tabelle 2) zeigen ebenfalls Signale im Rahmen der Fluoreszenzdetektion. Diese konnten jedoch zeitlich deutlich von den Proben unterschieden werden und wurden daher nicht berücksichtigt. Diese Signale in den Negativkontrollen sind das Ergebnis von unspezifischen Amplifikationen, beispielsweise Primerartefakten, da im Rahmen dieser Versuche der unspezifische Farbstoff "SYBR Green I" für die Detektion zum Einsatz kam. Ein an die jeweilige Anwendung angepasstes Primer-Screening in weiterführenden Versuchen ermöglicht es, optimale Primersequenzen für die jeweilige Zielanwendung auszuwählen.

Des Weiteren können anstelle von "SYBR Green I" spezifische Fluoreszenz-markierte Sonden verwendet werden, um Signale in den Negativkontrollen zu vermeiden.

Beispiel 4:

Es wurden sowohl vollständig vitale als auch inaktivierte E. *coli* Proben jeweils in Vierfachbestimmung mittels RPA analysiert und parallel die Lebendzellzahl durch Ausplattieren auf Nähragar in verschiedenen Verdünnungsstufen ermittelt (Bestimmung der Koloniezahlen nach einer Inkubationszeit von 24 h bei 37 °C).

Eine Standardkurve mit quantifizierter genomischer DNA und eine Negativkontrolle (Kontrolle ohne Template; Zugabe von "PCR-Grade"-Wasser anstatt der Probe) wurden parallel in der RPA mitlaufen gelassen. Alle 30 Sekunden über 30 min wurden Fluoreszenzdaten bei einer konstanten Reaktionstemperatur von 37 °C aufgezeichnet.

Ein 50 µl-Reaktionsansatz enthielt folgende Komponenten
Lyophilisiertes Pellet des RPA-Kits "TwistAmp fpg"
29,5 µl Puffer des RPA-Kits "TwistAmp fpg"
420 nM Primer mit der Sequenz:
5'-CAGGATTAGATACCCTGGTAGTCCACGCCGTAAAC-3' (SEQ ID NO: 1)
420 nM Primer mit der Sequenz:
5'-TAAGGGGCATGATGATTTGACGTCATCCCCACCTT-3' (SEQ ID NO: 2)
1 µl "SYBR Green I" (1:50.000 verdünnt)
13,2 µl Probe
12 mM Magnesiumacetat

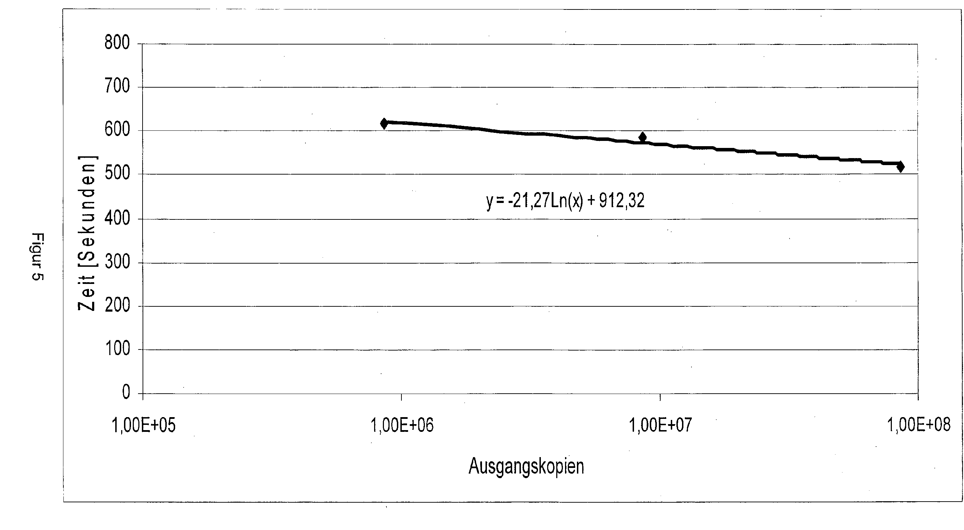

Die Standardkurve sowie die entsprechenden Werte sind in Figur 5 und Tabelle 3 gezeigt.

Tabelle 3: Werte der Standardkurve unter Verwendung quantifizierter E. *coli* DNA

| Quantifizierter DNA-Standard | Zeit bis Fluoreszenzzunahme [Sekunden] (Mittelwert, n=2) | Konzentration [Kopien/Reaktion] |
|---|---|---|
| E.coli Standard 1:10 verdünnt | 517 | $8{,}58 \times 10^7$ |
| E.coli Standard 1:100 verdünnt | 586 | $8{,}58 \times 10^6$ |
| E.coli Standard 1:1000 verdünnt | 615 | $8{,}58 \times 10^5$ |

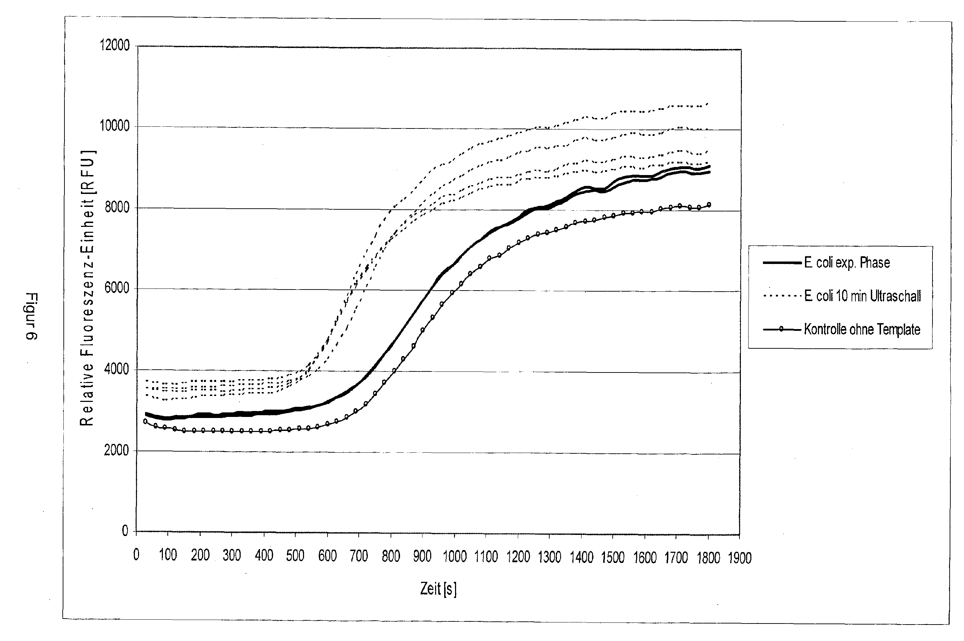

Die für die Proben erhaltenen Werte und Fluoreszenzkurven sind in Figur 6, sowie in Tabelle 4 gezeigt.

Tabelle 4: Ermittelte Konzentrationen der Genomeinheiten (Zellzahl) pro Reaktion bzw. pro ml

| Probe | Zeit bis Fl-zunahme [Sekunden] | Konzentration [Kopien/Reaktion] | Mittelwert Konzentration [Kopien/Reaktion] | Mittelwert Konzentration [Kopien/ml] |
|---|---|---|---|---|
| E. coli 10 min Ultraschall = GE$_{gesamt}$ | 526 | $7{,}87 \times 10^7$ | $4{,}60 \times 10^7$ | $3{,}48 \times 10^9$ |
| | 527 | $7{,}34 \times 10^7$ | | |
| | 549 | $2{,}66 \times 10^7$ | | |
| | 583 | $5{,}25 \times 10^6$ | | |
| E. coli exponentielle Phase = GE$_{tot}$ | 665 | $1{,}15 \times 10^5$ | $2{,}12 \times 10^5$ | $1{,}60 \times 10^7$ |
| | 636 | $4{,}32 \times 10^5$ | | |
| | 656 | $1{,}73 \times 10^5$ | | |
| | 662 | $1{,}27 \times 10^5$ | | |
| Kontrolle ohne Template | 677 | $6{,}53 \times 10^4$ | | |

Es ergibt sich für die Anzahl lebender Zellen in der Probe ($N_{lebend}$), bzw. für die entsprechende Anzahl von Genomeinheiten ($GE_{lebend}$):

$$GE_{lebend} = GE_{gesamt} - GE_{tot} = 3{,}48 \times 10^9 - 1{,}60 \times 10^7 = \underline{3{,}47 \times 10^9 \text{ GE/ml}}$$

Die Daten aus Tabelle 4 führen zu dem Ergebnis, dass die *E. coli* Kultur in der exponentiellen Phase $3{,}47 \times 10^9$ lebende Zellen pro Milliliter enthielt und lediglich $1{,}60 \times 10^7$ tote Bakterien, entsprechend 0,46%. Die zugehörigen Fluoreszenzkurven sind in Fig. 6 dargestellt.

Das parallele Ausplattieren auf Nähragar führte zu niedrigeren, jedoch plausiblen Ergebnissen, da davon auszugehen ist, dass nie 100% der lebenden Bakterien anwachsen und Kolonien bilden, da beispielsweise VBNC vorhanden sein können. In ein VBNC-Stadium gehen zahlreiche Bakterien über, wenn beispielsweise ungünstige Umgebungsbedingungen herrschen. Es kommt zum Herunterfahren des Stoffwechsels, so dass das Wachstum auf Standardnähragar nicht mehr möglich ist. Des Weiteren ist zu berücksichtigen, dass oft nicht eine Kolonie aus einem einzelnen Keim der Ausgangsprobe gewachsen ist, sondern auf mehrere, nicht vollständig separierte Mikroorganismen zurückzuführen ist. Durch Ausplattieren wurde eine Lebendzellzahl von *E. coli* in der exponentiellen Phase von etwa $5{,}0 \times 10^8$ Bakterien pro Milliliter ermittelt.

**Patentansprüche**

1. Verfahren zur quantitativen Bestimmung von lebenden und toten Zellen in einer biologischen Probe, umfassend die Schritte:

   (a) Bereitstellen eines unbehandelten Aliquots der Probe,
   (b) Bereitstellen eines Aliquots der Probe, in dem alle Zellen inaktiviert wurden, wobei die Zellen durch ein Verfahren inaktiviert wurden, das einerseits zu einem vollständigen Verlust der Integrität der Zellmembranen führt und andererseits die nachfolgenden Schritte nicht behindert,
   (c) Durchführen jeweils einer DNA-Amplifikationsreaktion mit den in den Schritten (a) und (b) bereitgestellten Aliquots, wobei die DNA-Amplifikationsreaktion die Membranintegrität lebender Zellen nicht beeinträchtigt und wobei die DNA-Amplifikationsreaktion eine isothermale oder nicht-isothermale Amplifikationsreaktion ist, welche bei Temperaturen unterhalb von 50 °C abläuft,
   (d) Bestimmen der jeweiligen DNA-Menge in den Aliquots anhand der in Schritt (c) durchgeführten DNA-Amplifikationsreaktionen, und
   (e) Bestimmen der Anzahl lebender und toter Zellen in der biologischen Probe aus den in Schritt (d) bestimmten DNA-Mengen.

2. Verfahren nach Anspruch 1, wobei die DNA-Amplifikationsreaktion eine isothermale Amplifikationsreaktion ist.

3. Verfahren nach Anspruch 1, wobei die DNA-Amplifikationsreaktion bei einer Temperatur zwischen 36 und 42 °C abläuft.

4. Verfahren nach einem der Ansprüche 1 bis 3, wobei die DNA-Amplifikationsreaktion eine Rekombinase-Polymerase-Amplifikation ist.

5. Verfahren nach einem der Ansprüche 1 bis 4, wobei bei der Inaktivierung der Zellen des in Schritt (b) bereitgestellten Aliquots die DNA dieser Zellen extrahiert wird, und in Schritt (c) diese DNA in der entsprechenden DNA-Amplifikationsreaktion eingesetzt wird.

6. Verfahren nach einem der Ansprüche 1 bis 5, wobei die Bestimmung der jeweiligen DNA-Menge in Schritt (d) unter Verwendung von Fluoreszenz-markierten Sonden oder DNA-bindenden Fluoreszenzfarbstoffen durchgeführt wird.

7. Verfahren nach Anspruch 6, wobei die Bestimmung der jeweiligen DNA-Menge in Schritt (d) mit Hilfe eines Fluorometers durchgeführt wird.

8. Verfahren nach Anspruch 7, wobei das Fluorometer ein Realtime-Fluorometer ist.

9. Verfahren nach einem der Ansprüche 1 bis 5, wobei die Bestimmung der jeweiligen DNA-Menge in Schritt (d) mit Hilfe einer Gelelektrophorese und anschließendem Vergleich der Bandenstärken durchgeführt wird.

10. Verfahren nach einem der Ansprüche 1 bis 9, wobei die Bestimmung der Anzahl lebender und toter Zellen in der biologischen Probe in Schritt (e) mit Hilfe einer Standardkurve durchgeführt wird, welche durch DNA-Amplifikationsreaktionen mit parallelen Reaktionsansätzen erhalten wird, die bekannte DNA-Konzentrationen enthalten.

11. Verfahren nach einem der Ansprüche 1 bis 10, wobei die Bestimmung der Anzahl lebender Zellen in der biologischen Probe in Schritt (e) anhand der Formel $N_{lebend} = N_{gesamt} - N_{tot}$ erfolgt, wobei $N_{gesamt}$ anhand der in Schritt (d) bestimmten DNA-Menge in dem in Schritt (b) bereitgestellten Aliquot, und $N_{tot}$ anhand der in Schritt (d) bestimmten DNA-Menge in dem in Schritt (a) bereitgestellten Aliquot bestimmt wird.

12. Verfahren nach einem der Ansprüche 1 bis 11, wobei die Zellen prokaryotische Zellen sind.

**Claims**

1. Method for the quantitative determination of living and dead cells in a biological sample, comprising the steps of:

   a) providing an untreated aliquot of the sample,
   b) providing an aliquot of the sample in which all cells have been inactivated, wherein the cells have been inactivated by means of a method which on the one hand leads to a complete loss of integrity of the cell membranes and on the other hand does not impede the subsequent steps,
   c) carrying out a respective DNA amplification reaction with the aliquots provided in the steps (a) and (b), wherein the DNA amplification reaction does not affect the membrane integrity of living cells and wherein the DNA amplification reaction is an isothermal or non-isothermal amplification reaction taking place at temperatures below 50°C,
   d) determining the respective amount of DNA in the aliquots by means of the DNA amplification reactions carried out in step (c), and
   e) determining the number of living and dead cells in the biological sample from the amounts of DNA determined in step (d).

2. Method according to claim 1, wherein the DNA amplification reaction is an isothermal amplification reaction.

3. Method according to claim 1, wherein the DNA amplification reaction takes place at a temperature between 36 and 42°C.

4. Method according to any of the claims 1 to 3, wherein the DNA amplification reaction is a Recombinase Polymerase Amplification.

5. Method according to any of the claims 1 to 4, wherein during the inactivation of the cells of the aliquot provided in step (b) the DNA of these cells is extracted, and in step (c) this DNA is used in the corresponding DNA amplification reaction.

6. Method according to any of the claims 1 to 5, wherein the determination of the respective amount of DNA in step (d) is carried out using fluorescence-marked probes or DNA-binding fluorescent dye.

7. Method according to claim 6, wherein the determination of the respective amount of DNA in step (d) is carried out by means of a fluorometer.

8. Method according to claim 7, wherein the fluorometer is a realtime fluorometer.

9. Method according to any of the claims 1 to 5, wherein the determination of the respective amount of DNA in step (d) is carried out by means of a gel electrophoresis and subsequent comparison of the size of the bands.

10. Method according to any of the claims 1 to 9, wherein the determination of the number of living and dead cells in the biological probe in step (e) is carried out by means of a standard curve obtained from DNA amplification reactions with parallel reaction batches containing known concentrations of DNA.

11. Method according to any of the claims 1 to 10, wherein the determination of the number of living cells in the biological probe in step (e) is carried out by means of the formula $N_{living} = N_{total} - N_{dead}$, wherein $N_{total}$ is determined by means of the amount of DNA determined in step (d) in the aliquot provided in step (b), and $N_{dead}$ is determined by means of the amount of DNA determined in step (d) in the aliquot provided in step (a).

12. Method according to any of the claims 1 to 11, wherein the cells are prokaryotic cells.

**Revendications**

1. Procédé pour la détermination quantitative de cellules vivantes et mortes dans un échantillon biologique, comprenant les étapes :

   (a) fourniture d'une aliquote non traitée de l'échantillon,

(b) fourniture d'une aliquote de l'échantillon dans laquelle toutes les cellules ont été inactivées, les cellules ayant été inactivées par un procédé qui d'une part conduit à une perte complète de l'intégrité des membranes cellulaires et d'autre part n'empêche pas les étapes suivantes,

(c) mise en oeuvre d'une réaction d'amplification de l'ADN à l'aide de chacune des aliquotes fournies dans les étapes (a) et (b), la réaction d'amplification de l'ADN n'influençant pas l'intégrité membranaire des cellules vivantes, et la réaction d'amplification de l'ADN étant une réaction d'amplification isotherme ou non isotherme, qui se déroule à des températures inférieures à 50°C,

(d) détermination de la quantité d'ADN dans chaque aliquote à l'aide des réactions d'amplification de l'ADN mises en oeuvre dans l'étape (c), et

(e) détermination du nombre de cellules vivantes et mortes dans l'échantillon biologique à partir des quantités d'ADN déterminées dans l'étape (d).

2. Procédé selon la revendication 1, dans lequel la réaction d'amplification de l'ADN est une réaction d'amplification isotherme.

3. Procédé selon la revendication 1, dans lequel la réaction d'amplification de l'ADN se déroule à une température comprise entre 36 et 42°C.

4. Procédé selon l'une des revendications 1 à 3, dans lequel la réaction d'amplification de l'ADN est une amplification par recombinase-polymérase.

5. Procédé selon l'une des revendications 1 à 4, dans lequel, lors de l'inactivation des cellules de l'aliquote fournie dans l'étape (b), l'ADN de ces cellules est extrait, et on utilise dans l'étape (c) cet ADN dans la réaction d'amplification de l'ADN correspondante.

6. Procédé selon l'une des revendications 1 à 5, dans lequel la détermination de chaque quantité d'ADN dans l'étape (d) est mise en oeuvre par utilisation de sondes marquées par fluorescence ou de colorants fluorescents liant l'ADN.

7. Procédé selon la revendication 6, dans lequel ladite détermination de chaque quantité d'ADN dans l'étape (d) est mise en oeuvre à l'aide d'un fluoromètre.

8. Procédé selon la revendication 7, dans lequel le fluoromètre est un fluoromètre en temps réel.

9. Procédé selon l'une des revendications 1 à 5, dans lequel la détermination de chaque quantité d'ADN dans l'étape (d) est mise en oeuvre à l'aide d'une électrophorèse sur gel, suivie d'une comparaison des intensités des bandes.

10. Procédé selon l'une des revendications 1 à 9, dans lequel la détermination du nombre de cellules vivantes et mortes dans l'échantillon biologique dans l'étape (e) est mise en oeuvre à l'aide d'une courbe étalon, qui est obtenue par des réactions d'amplification de l'ADN utilisant des masses réactionnelles en parallèle, qui contiennent des concentrations connues d'ADN.

11. Procédé selon l'une des revendications 1 à 10, dans lequel la détermination du nombre de cellules vivantes dans l'échantillon biologique dans l'étape (e) est effectuée à l'aide de la formule $N_{vivantes} = N_{totales} - N_{mortes}$, où $N_{totales}$ est déterminé à l'aide de la quantité d'ADN déterminée dans l'étape (d) dans l'aliquote fournie dans l'étape (b), et $N_{mortes}$ est déterminé à l'aide de la quantité d'ADN déterminée dans l'étape (d) dans l'aliquote fournie dans l'étape (a).

12. Procédé selon l'une des revendications 1 à 11, dans lequel les cellules sont des cellules procaryotes.

Figur 1

Figur 2

Figur 3

Figur 4

Figur 5

Figur 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007100762 A2
- EP 1992690 A1
- US 20090123959 A1
- WO 2011043737 A1
- WO 2011068465 A1
- WO 1996014430 A1
- US 6146834 A
- WO 1999014359 A1
- WO 2002052034 A1
- JP 2001299399 A
- DE 102008029999 A1
- DE 102012014981

**In der Beschreibung aufgeführte Nicht-Patentliteratur**

- **CHEN et al.** *APPLIED AND ENVIRONMENTAL MICROBIOLOGY,* 2011, vol. 77 (12), 4008-4016